# EUROPÄISCHE PATENTANMELDUNG

(11) **EP 0 992 237 A2**
(43) Veröffentlichungstag der Anmeldung: **12.04.2000**
(21) Anmeldenummer: 99117983.9
(22) Anmeldetag: 17.09.1999
(51) Int. Cl.: A61K 9/70, A61K 47/26

(54) **Neohesperidin dc als Kristallisationsinhibitor**

(30) Priorität: 19.09.1998 DE 19843027
(71) Anmelder: LABTEC GESELLSCHAFT FüR TECHNOLOGISCHE FORSCHUNG UND ENTWICKLUNG MBH, 40764 Langenfeld (DE)
(72) Erfinder: Cordes, Günter, Dr., 40764 Langenfeld (DE); Vollmer, Ulrike, Dr., 40764 Langenfeld (DE); Mertin, Dirk, Dr., 40764 Langenfeld (DE)
(74) Vertreter: König, Beate, Dipl.-Phys. Dr.

(57) **Zusammenfassung**

Transdermale therapeutische Systeme, welche Neohesperidin-Dihydrochalkon als Kristallisationsinhibitor enthalten.

## Beschreibung

Neohesperidin-Dihydrochalkon (Neohesperidin DC) ist ein Flavonglykosid, welches in der Schale der Grapefruit (Citrus aurantium L. ssp. aurantiurn) vorkommt und durch süßen Geschmack gekennzeichnet ist [Inglett et al., J Food Sci 34, 101 (1969)]. Es hat eine etwa 1000 bis 1500 mal stärkere Süßkraft als Saccharose. Aufgrund der toxikologischen Unbedenklichkeit wurde die Substanz inzwischen als Süßstoff für Lebensmittel erlaubt (E 959; Zusatzstoff-Zulassungsverordnung, Anlage 2B).

Die Verwendung zu anderen als zu Süßungszwecken ist hingegen weniger bekannt. DE 19646050 beschreibt den Einsatz in einem transdermalen therapeutischen System. Danach soll die Substanz durch Hemmung oxidativer Enzyme (Cytochrom P 450) den Metabolismus der applizierten Wirkstoffe hemmen und somit deren Bioverfügbarkeit verbessern.

Die Penetration von Arzneistoffen durch die Haut ist weitgehend durch die physikochemischen Eigenschaften der Substanz bestimmt. Hierbei spielen im wesentlichen der Octanol/Wasser-Verteilungskoeffizient sowie die Molekülgröße eine Rolle (Potts RO, Guy RH in: Gurny R, Teubner A; Dermal and transdermal drug delivery, Wiss. Verlagsges. Stuttgart (1993)). Da diese Parameter ohne Molekülmodifikationen nicht beeinflußbar sind, gibt es im wesentlichen nur zwei Möglichkeiten, die Penetrationsrate zu steigern:
1. Erleichterung der Diffusion durch Zusatz von Penetrationsbeschleunigern oder Anwendung von elektrischer Spannung (Iontophorese)
2. Steigerung der Arzneistoffkonzentration in der Grundlage auch über die Löslichkeitsgrenze hinaus (Übersättigung).

Als Penetrationsbeschleuniger werden u.a. Fettsäuren, Fettalkohole, einfache und mehrwertige Alkohole, Laurocapram und Tenside eingesetzt. Viele dieser Substanzen wirken jedoch über eine Störung der Barrierefunktion der Haut und sind damit als mehr oder weniger hautreizend einzustufen.

Verträglicher ist die Verwendung von Systemen, bei denen der Wirkstoff in übersättigter Form vorliegt. Üblicherweise ist der maximale Flux einer Substanz durch die Haut durch seine Löslichkeit in der Hornhaut (Stratum corneum), welche die Hauptpenetrationsbarriere darstellt, begrenzt. Diese Sättigungskonzentration wird sich dann einstellen, wenn der Wirkstoff im Vehikel, z.B. in der Matrix des Transdermalsystems, ebenfalls in einer Konzentration vorliegt, die der Löslichkeit im Vehikel entspricht. Eine Möglichkeit, diese sog. maximale thermodynamische Aktivität weiter zu erhöhen, besteht darin, den Arzneistoff in einer die Löslichkeit im Vehikel überschreitenden Konzentration einzuarbeiten.

Solche übersättigten Systeme haben jedoch den entscheidenden Nachteil, daß sie metastabil sind und, abhängig von den Lagerbedingungen, dem Grad der Übersättigung und der Viskosität der Zubereitung nach einer gewisser Zeit zur Wirkstoffrekristallisation neigen. Diese Kristallisation tritt in der Regel erst während der Lagerung des Produktes auf, nur bei starker Übersättigung auch direkt nach Herstellung. Sie ist einerseits unerwünscht, da hierdurch die für die Penetration zur Verfügung stehende, freie Wirkstoffmenge und damit letztendlich die Bioverfügbarkeit reduziert wird. Andererseits führt sie, sofern für den Verbraucher erkennbar, zur Reklamation bzw. Ablehnung des Produktes.

Zur Verhinderung der Rekristallisation bzw. des Kristallwachstums in wäßrigen Systemen ist der Zusatz von Schutzkolloiden (Gelatine, PVP), Netzmitteln und Peptisatoren (Citrat, Phosphat) bekannt (Sucker H, Fuchs P, Speiser P; Pharmazeutische Technologie, S. 561f., G. Thieme Verlag Stuttgart (1991)). In polymeren Matrices, die für die Herstellung von transdermalen Systemen (TDS) häufig verwendet werden, führen derartige Maßnahmen allerdings nicht immer zum Erfolg. Ma et al. fanden jedoch einen positiven Einfluß von PVP auf die Kristallisation von NETA in einem Acrylat-Matrixpflaster (Ma X, Taw J, Chiang CM; Int J Pharm 142, 115-119 (1996)), während kolloidale Kieselsäure und Cholesterol als Kristallisationsinhibitoren für Estradiol in Acrylatklebern beschrieben wurden (DE 4210711). Es ist jedoch dem Fachmann bekannt, daß derartige Zusätze, insbesondere in höheren Konzentrationen, die Klebeeigenschaften des Pflasters nachteilig verändern können.

Überraschend wurde nun gefunden, daß der Süßstoff Neohesperidin DC die Rekristallisation von Wirkstoffen in transdermalen therapeutischen Systemen hemmt, und auch bereits in niedriger Konzentration (bis ca. 5%). Die zu diesem Zweck eingesetzte Menge Neohesperidin DC verändert im Gegensatz zu den oben beschriebenen Kristallisationsinhibitoren nicht die Klebeeigenschaften der Pflastersysteme (Tabelle 1).

Die erfindungsgemäßen transdermalen therapeutischen Systeme sind vorzugsweise so beschaffen, daß sie aus einer für den Wirkstoff undurchlässigen Deckschicht, einer auf der Deckschicht haftenden wirkstoffhaltigen Kleberschicht, welche auch Neohesperidin DC enthält, und einer abziehbaren Schutzschicht bestehen.

Diese einfachste Form eines TDS kann in der Weise hergestellt werden, daß man eine Lösung des Klebers in einem niedrigsiedendem Lösungsmittel mit dem Wirkstoff und Neohesperidin DC mischt, die Mischung gleichmäßig auf einer abziehbaren Schutzschicht aufträgt, das Lösungsmittel durch Erwärmen entfernt und das erhaltene Produkt mit einer Deckschicht abdeckt.

Als Schutzschicht eignen sich alle Folien, die man üblicherweise bei TDS anwendet, insbesondere silikon- oder fluorpolymerbeschichtete Folien. Als Deckschicht kann man bei diesen Systemen beispielsweise 10 bis 100 µm dicke Folien aus PVC, PVDC, EVA, Polyethylen oder Polyester sowie deren Copolymerisate verwenden. Die hierauf aufgebrachte wirkstoffhaltige Kleberschicht hat vorzugsweise eine Dicke von 20 bis 500 µm. Grundlage des Klebers sind Polyacrylate, Silikone oder natürliche bzw. synthetische Kautschuke.

Es ist für den Fachmann offenkundig, daß die erfindungsgemäßen TDS auch wesentlich komplexer als die bereits erwähnten einfachen Matrixsysteme sein können.

Die nachfolgenden Ausführungsbeispiele dienen zur näheren Erläuterung der Erfindung:

### Beispiel 1:

Zu 14,92 g einer 51 %igen (m/m) Lösung eines Acrylat-Klebstoffes (Durotak 337-2287, National Starch & Chemical B.V., NL-Zutphen) wird eine Lösung von 0,48 g Testosteron, 0,44 g Ölsäure und 0,26 g Neohesperidin DC in 6,4 g Ethanol 96% gegeben. Durch einstündiges Rühren wird die Lösung homogenisiert und anschließend mit einem Rakel auf einer silikonisierten, 100 µm starken Polyesterfolie (FL 2000 100 µ 1-S, Rexam Release B.V., NL-Apeldoorn) in einer Naßschichtdicke von 360 µm ausgestrichen. Nach der Trocknung (1 h bei 40°C und 10 min bei 80°C) wird das leicht trübe Laminat mit einer Polyesterfolie (Hostaphan RN 15, Hoechst Diafoil GmbH, D-Wiesbaden) kaschiert.

### Beispiel 2:

Zu 14,06 g einer 51 %igen (m/m) Lösung eines Acrylat-Klebstoffes (Durotak 387-2287, National Starch & Chemical B.V., NL-Zutphen) wird eine Lösung von 0,48 g Testosteron, 0,88 g Ölsäure und 0,26 g Neohesperidin DC in 6,4 g Ethanol 96% gegeben. Durch einstündiges Rühren wird die Lösung homogenisiert und anschließend mit einem Rakel auf einer silikonisierten, 100 µm starken Polyesterfolie (Akrosil NAT 100m PET Silox H2A/H2A, International Paper Div. Akrosil, US-Menasha) in einer Naßschichtdicke von 360 µm ausgestrichen. Nach der Trocknung (1 h bei 40°C und 10 min bei 80°C) wird das leicht trübe Laminat mit einer Polyesterfolie (Hostaphan RN 15, Hoechst Diafoil GmbH, D-Wiesbaden) kaschiert.

### Beispiel 3:

Zu 19,84 g einer 51 %igen (m/m) Lösung eines Acrylat-Klebstoffes (Durotak 387-2287, National Starch & Chemical B.V., NL-Zutphen) wird eine Lösung von 0,64 g Testosteron, 0,16 g Estradiol-Hemihydrat, 0,19 g Levonorgestrel, 0,29 g Ölsäure und 0,35 g Neohesperidin DC in einer Mischung aus 6,0 g Methylethylketon, 0,29 g Transcutol und 4,3 g Ethanol 96% gegeben. Durch einstündiges Rühren wird die Lösung homogenisiert und anschließend mit einem Rakel auf einer silikonisierten, 100 µm starken Polyesterfolie (FL 2000 100 µ 1-S, Rexam Release B.V., NL-Apeldoorn) in einer Naßschichtdicke von 420 µm ausgestrichen. Nach der Trocknung (1 h bei 40°C) wird das leicht trübe Laminat mit einer Polyethylenfolie (Cotran 9720, 3M Medica, D-Borken) kaschiert.

### Beispiel 4:

Zu 17,46 g einer 35 %igen (m/m) Lösung eines Acrylat-Klebstoffes (Durotak 87-2852, National Starch & Chemical B.V., NL-Zutphen) wird eine Lösung von 2,08 g Ketoprofen und 0,21 g Neohesperidin DC in 4,17 g Isopropanol gegeben. Durch einstündiges Rühren wird die Lösung homogenisiert und anschließend mit einem Rakel auf einer silikonisierten, 100 µm starken Polyesterfolie (FL 2000 100 µ 1-S, Rexam Release B.V., NL-Apeldoorn) in einer Naßschichtdicke von 270 µm ausgestrichen. Nach der Trocknung (1 h bei 40°C) wird das leicht trübe Laminat mit einem längs- und querelastischen Polyestergewebe (K. O. Braun, D-Wolfstein) kaschiert. Ein Pflaster der Größe 90 cm² enthält bei einem Matrixgewicht von 55,6 g/m² 125 mg Ketoprofen.

Wie Tabelle 2 zeigt, weisen alle Beispiele gegenüber den entsprechenden Rezepturen ohne Neohesperidin DC eine reduzierte Kristallisation auf.

**Tabelle 1**

| *Beeinflussung der Klebeeigenschaften von transdermalen therapeutischen Systemen durch Neohesperidin DC (Beispiel 4, n=3)* | | |
|---|---|---|
| | Ketoprofen TDS ohne Zusatz von Neohesperidin DC | Ketoprofen TDS mit Zusatz von 2,5% Neohesperidin DC (bezogen auf die Matrix) |
| Klebkraft [N/25 mm] | 6,8 ± 0,6 | 6,2 ± 0,4 |
| Trennkraft [N/25 mm] | 0,137 ± 0,012 | 0,127 ± 0,025 |

**Tabelle 2**

| *Rekristallisation pharmazeutischer Wirkstoffe in transdermalen therapeutischen Systemen mit und ohne Zusatz von Neohesperidin DC* | | | | | |
|---|---|---|---|---|---|
| Zusammensetzung | Lagerbedingung | | | | |
| | Raumtemp., offen | 2 5°C/60% rF, offen | 25°C/60% rF, versieg. | 40°C/75% rF, offen | 40°C/75% rF, versieg. |
| Beispiel 1 | -¹ | n.b. | n.b. | n.b. | n.b. |
| wie Beispiel 1, aber ohne Neohesperidin DC | ++¹ | n.b. | n.b. | n.b. | n.b. |
| Beispiel 2 | -¹ | n.b. | n.b. | n.b. | n.b. |
| wie Beispiel 2, aber ohne Neohesperidin DC | ++¹ | n.b. | n.b. | n.b. | n.b. |
| Beispiel 3 | n.b. | -² | -² | +² | -² |
| wie Beispiel 3, aber ohne Neohesperidin DC | n.b. | ++² | +² | ++² | ++² |
| Beispiel 4 | -³ | n.b. | +³ | n.b. | +³ |
| wie Beispiel 4, aber ohne Neohesperidin DC | +³ | n.b. | ++³ | n.b. | ++³ |
| - keine, + geringe, ++ starke Rekristallisation des/der Wirkstoffe(s) n.b.: nicht bestimmt | | | | | |

| | | | | | |
|---|---|---|---|---|---|
| ¹ nach 1 Tag Lagerung | | | | | |
| ² nach 8 Wochen Lagerung | | | | | |
| ³ nach 6 Monaten Lagerung | | | | | |

## Patentansprüche

1. Transdermales therapeutisches System, dadurch gekennzeichnet, daß es in einer wirkstoffübersättigten Klebermatrix Neohesperidin DC als Kristallisationsinhibitor enthält.

2. Transdermales therapeutisches System nach Anspruch 1, dadurch gekennzeichnet, daß es Neohesperidin DC in einer Konzentration von 0,1 bis 10%, bezogen auf die Matrix, vorzugsweise 1 bis 5% enthält.

3. Transdermales therapeutisches System nach Anspruch 1 bis 2, dadurch gekennzeichnet, daß es als Wirkstoff ein oder mehrere Sexualhormone enthält.

4. Transdermales therapeutisches System nach Anspruch 1 bis 3, dadurch gekennzeichnet, daß es als Wirkstoff Testosteron, allein oder in Kombination mit anderen Sexualhormonen, enthält.

5. Transdermales therapeutisches System nach Anspruch 1 bis 2, dadurch gekennzeichnet, daß es als Wirkstoff ein nichtsteroidales Antirheumatikum enthält.

6. Transdermales therapeutisches System nach Anspruch 5, dadurch gekennzeichnet, daß es als Wirkstoff Ketoprofen enthält.
